# EUROPÄISCHE PATENTSCHRIFT

(11) **EP 4 175 617 B1**
(45) Veröffentlichungstag und Bekanntmachung des Hinweises auf die Patenterteilung: **27.08.2025**
(21) Anmeldenummer: 21756033.3
(22) Anmeldetag: 01.07.2021
(51) Int. Cl.: A61K 9/00, A61K 47/12, A61K 47/26, A61K 36/53, A61P 1/00, A61K 9/68

(54) **ZUSAMMENSETZUNG VERWENDET ALS STIMMUNGSREGULIERENDES STIMULANS**
COMPOSITION USED AS A MOOD-REGULATING STIMULANT
COMPOSITION UTILISÉE COMME STIMULANT DESTINÉ À RÉGULER L'HUMEUR

(30) Priorität: 01.07.2020 DE 102020117395
(43) Veröffentlichungstag der Anmeldung: 10.05.2023
(73) Patentinhaber: Happygum G.m.b.H., 1010 Wien (AT)
(72) Erfinder: FARKAS, Tim, 1010 Wien (AT)
(74) Vertreter: von Bülow & Tamada
(86) Internationale Anmeldenummer: PCT/IB2021/055934
(87) Internationale Veröffentlichungsnummer: WO 2022/003630

(56) Entgegenhaltungen:
- EP-A1- 1 623 716
- EP-A1- 2 219 467

## Beschreibung

Die vorliegende Erfindung betrifft eine Zusammensetzung verwendet als stimmungsregulierendes Stimulans.

Aus Uehleke, B., et al., "Phase II trial on the effects of Silexan in patients with neurasthenia, post-traumatic stress disorder or somatization disorder", Phytomedicine, Jahr 2012, Ausgabe 19, Seite 665 bis 671 ist bekannt, Lavendel als Antidepressiva mit sedierender und anxiolytischer Wirkung und daher auch als Beruhigungsmittel einzusetzen.

Die EP1623716 A1 offenbart einen ein Kaugummi, der Lavendelöl enthält, als ein Beispiel für eine Darreichungsform von Arzneimitteln. Dieser Kaugummi wird als eine mögliche Form der Anwendung von Lavendelöl, zur Behandlung von stressbedingten Erkrankungen, Syndromen und Leiden, die durch geistige Überbeanspruchung und Stress verursacht werden dargestellt.

Die EP2219467A1 offenbart Kaugummi-Kompositionen, die aktive Inhaltsstoffe wie Koffein enthalten.

Es ist Aufgabe der Erfindung, eine weitere Verwendung von Lavendel anzugeben.

Gemäß einem Aspekt der Erfindung ist eine Zusammensetzung zusammengesetzt aus Lavendelöl als Wirkstoff zur medizinischen Verwendung bei einer Behandlung einer Konzentrationsstörung oder Aufmerksamkeitsstörung (ADS) und einer Kaumasse, dadurch gekennzeichnet, dass die Zusammensetzung die Kaumasse umfasst und Lavendelöl als Wirkstoff in einer Konzentration von 0,1 bis 1 Gew.%, bevorzugt von 0,2 bis 0,4 Gew.%, besonders bevorzugt von 0,3 Gew% liegt, wobei die Kaumasse eine Zugfestigkeit zwischen 0,25 N/mm² und 4 N/ mm zur Beanspruchung der Gesichtsmuskeln aufweist.

Anhand von Versuchen hat sich herausgestellt, dass Lavendelöl auch als Stimulans einsetzbar ist. Die Wirkung ist daher zu einem Antidepressiva mit sedierender und anxiolytischer Wirkung umgekehrt, aktiviert die Kognition und vertreibt Müdigkeit.

Allerdings zeigt Lavendel oral eingenommen unerwünschte Effekte, wie Magen-Darm-Störungen in Form von Dyspepsie und Übelkeit. Auch sind allergische Reaktionen bekannt.

Gerade gegen Konzentrationsstörungen neigen Anwender dazu, einen potentiellen Wirkstoff über den Tag gesehen häufig und in hohen Dosen einzunehmen, was die zu vor genannten unerwünschten Effekte weiter steigert.

Zur Anwendung von Lavendel als stimmungsregulierendes Stimulans schlägt die vorliegende Erfindung daher eine Verabreichung in Kaugummiform vor, so dass das Lavendelöl bukkal oder sublingual aufgenommen wird. Hier konnte durch Versuche nachgewiesen werden, dass sich die oben genannte kognitionssteigernde und Müdigkeit vertreibende Wirkung schon mit einer deutlich geringeren Menge an Lavendel verglichen mit anderen Darreichungsformen, wie Kapseln oder Tabletten erreichen lässt.

In einer Weiterbildung der Zusammensetzung ist das Lavendelöl der einzige Wirkstoff.

Erfindungsgemäß weist die Kaumasse eine Zugfestigkeit zwischen 0,25 N/mm² und 4 N/mm² zur Beanspruchung der Gesichtsmuskeln auf.

In einer noch anderen Weiterbildung weist die Kaumasse als Konzistenzbildner synthetischen und/oder natürlichen Gummi auf.

In einer Weiterbildung des angegebenen Kaugummis beträgt der Anteil an Lavendelöl zwischen 0,2 Gew% und 0,4 Gew%. Bei der bukkalen und sublingualen Verabreichung gelangt eine deutlich höhere Konzentration an Lavendelöl in den Blutkreislauf, der durch das Kauen zudem noch angeregt wird. Bei einem Anteil an Lavendelöl von 0,3 Gew% lässt sich eine optimale Wirkung erzielen.

In einer zusätzlichen Weiterbildung der angegebenen Erfindung umfasst die angegebene Zusammensetzung ferner 0,05 bis 0,5 Gew%, vorzugsweise 0,08 bis 0,2 Gew %, besonders bevorzugt 0,1 Gew% eines Spurenelementes. Insbesondere das Spurenelement Zink unterstützt die Regeneration der Mundschleimhäute und stellt bei einer längerfristigen Einnahme der angegebenen Zusammensetzung sicher, dass das beim Kauen der Kaugummimasse freigegebene Lavendelöl optimal über die Mundschleimhäute aufgenommen werden kann.

In einer zusätzlichen Weiterbildung der angegebenen Erfindung umfasst die angegebene Zusammensetzung 0,1 Gew% bis 1 Gew %, vorzugsweise 0,15 Gew% bis 0,35 Gew%, besonders bevorzugt 0,25 Gew% eines Salzes. Salze wie Natriumchlorid Salz wirken antibakteriell, desinfizierend, lindernd, abschwellend und entzündungshemmend auf die Mundschleimhaut. So werden Bakterien im Mundraum reduziert und Entzündungen und Infektionen entgegengewirkt. Auch feuchten Salze bei einer Entzündung des Rachenraumes die gereizten und ausgetrockneten Schleimhäute an, die dadurch in ihrer Funktion unterstützt werden. Zudem besitzen Salze eine reinigende und durchblutungsfördernde Wirkung. Beläge und Schleim werden von den Schleimhäuten gelöst, sodass eine Regeneration des Zahnfleischs eintritt. Auf diese Weise wird die Aufnahme des Lavendelöls über die Mundschleimhäute weiter verbessert, kann optional.

In einer weiteren Weiterbildung umfasst die angegebene Zusammensetzung ferner umfassend 0,05 Gew% bis 0,5 Gew%, vorzugsweise 0,09 Gew% bis 0,3 Gew.%, besonders bevorzugt 0,15 Gew % eines Vitamin B. Vitmin B, wie zum Beispiel Pantothensäure und Niacin tragen zum Erhalt der Mundschleimhäute bei und stellen so die wirkungsvolle Aufnahme des Lavendelöl bei einer längerfristigen Einnahme der angegebenen Zusammensetzung sicher.

In einer noch weiteren Weiterbildung umfasst die angegebene Zusammensetzung ferner 0,1 Gew% bis 1 Gew%, vorzugsweise 0,2 Gew % bis 0,4 Gew %, besonders bevorzugt 0,3 Gew% eines Vitamin D. Vitamin D wie Biotin trägt zum Erhalt der Mundschleimhäute bei und stellt so die wirkungsvolle Aufnahme des Lavendelöl bei einer längerfristigen Einnahme der angegebenen Zusammensetzung sicher.

In einer besonderen Weiterbildung umfasst die angegebene Zusammensetzung ferner einen Säureregulator. Mit dem Säureregluator lässt sich die Speichelgeneration im Mundraum einstellen und so die Bedingung zur Aufnahme des Lavendelöls über die Mundschleimhäute weiter verbessern. Hierzu hat sich Zitronensäure als Säureregulator am geeignetsten erwiesen.

In einer anderen Weiterbildung der angegebenen Zusammensetzung umfasst die Kaumasse zwischen 50 Gew% und 99 Gew%, vorzugsweise zwischen 70 Gew% und 80 Gew%, besonders bevorzugt 75 Gew% eines Zuckeralkohols oder einer Mischung aus Zuckeralkoholen, wie Sorbit, Mannit, Isomalt, Maltit, Lactit, Xylit, Erythrit, Polyglycitolsirup. Zuckeralkohole haben eine leicht kühlende Wirkung im Mundraum, was sich ebenfalls positiv auf die bukkale oder sublinguale Aufnahme des Lavendelöls auswirkt.

Die oben beschriebenen Eigenschaften, Merkmale und Vorteile dieser Erfindung sowie die Art und Weise wie diese erreicht werden, werden verständlicher im Zusammenhang mit der folgenden Beschreibung der Ausführungsbeispiele, die im Zusammenhang mit der Zeichnung näher erläutert werden. Es zeigen:
Fig. 1 ein Diagramm mit über die Zeit aufgetragenen Mittelwerten zu positiv konnotierten Stimmungen von Probanden, die eine erfindungsgemäße Zusammensetzung eingenommen haben, gemäß dem Brief Mood Introspection Scale, kurz BMIS, 3rd edition, February 26, 2019,
Fig. 2 ein Diagramm mit über die Zeit aufgetragenen Mittelwerten zu positiv konnotierten Stimmungen von Probanden, die eine alternative Zusammensetzung eingenommen haben, gemäß dem Brief Mood Introspection Scale, kurz BMIS, 3rd edition, February 26, 2019,
Fig. 3 ein Diagramm mit über die Zeit aufgetragenen Mittelwerten zu negativ konnotierten Stimmungen von Probanden, die eine erfindungsgemäße Zusammensetzung eingenommen haben, gemäß dem Brief Mood Introspection Scale, kurz BMIS, 3rd edition, February 26, 2019,
Fig. 4 ein Diagramm mit über die Zeit aufgetragenen Mittelwerten zu negativ konnotierten Stimmungen von Probanden, die eine alternative Zusammensetzung eingenommen haben, gemäß dem Brief Mood Introspection Scale, kurz BMIS, 3rd edition, February 26, 2019,
Fig. 5 ein Diagramm mit einer Auswertung erhobener Versuchsdaten auf der "Pleasant-Unpleasant"-Skala gemäß dem Brief Mood Introspection Scale, kurz BMIS, 3rd edition, February 26, 2019,
Fig. 6 ein Diagramm mit einer Auswertung erhobener Versuchsdaten auf der "Excited-Calm"-Skala gemäß dem Brief Mood Introspection Scale, kurz BMIS, 3rd edition, February 26, 2019,
Fig. 7 ein Diagramm mit einer Auswertung erhobener Versuchsdaten auf der "Positive-Tired"-Skala gemäß dem Brief Mood Introspection Scale, kurz BMIS, 3rd edition, February 26, 2019, und
Fig. 8 ein Diagramm mit einer Auswertung erhobener Versuchsdaten auf der "Negative-Relaxed"-Skala gemäß dem Brief Mood Introspection Scale, kurz BMIS, 3rd edition, February 26, 2019.

In den Figuren werden gleiche technische Elemente mit gleichen Bezugszeichen versehen und nur einmal beschrieben.

Die Wirksamkeit der beanspruchten Zusammensetzung wurde mit einem Ausführungsbeispiel der beanspruchten Zusammensetzung in einem klinischen Versuch ermittelt und einer alternativen Zusammensetzung gegenübergestellt.

Das Ausführungsbeispiel der beanspruchten Zusammensetzung waren eine Vielzahl von Kaugummis von je 2g. Jeder Kaugummi enthielt 1,5g einer Zuckeralkoholmischung aus Sorbitol, Maltiol und Mannitol, eine Vitamin- und Mineralmischung aus 5mg Salz, 2mg Zink, 2mg Niacin, 0,8mg Pantothensäure und 6,5µg Biotin. Ferner war der Zusammensetzung noch 2mg Zitronensäure und 6mg Lavendelöl beigemischt. Der Rest jedes Kaugummies waren Weichmacher, Feuchthaltemittel, Antioxidantien, Aromen, Farbstoffe und Emulgatoren, die zur beanspruchten Wirkung der Zusammensetzung nichts beitragen.

Die alternative Zusammensetzung waren eine Vielzahl von Tabletten. Jede Tablette enthielt 50mg Lavendelblütenextrakt, 10mg Lavendelöl, 1,1 mg Vitamin B1, 1,4mg Vitamin B2, 6mg Pantothensäure, 1,4mg Vitamin B6 und 5µg Vitamin B12, wie sie beispielsweise von der Firma Queisser Pharma GmbH & Co. KG unter dem Handelsnahmen Doppelherz aktiv Lavendel erhältlich sind.

Jede Tablette der alternativen Zusammensetzung enthielt daher ungefähr das doppelte an Lavendelextrakt verglichen mit einem Kaugummi gemäß dem Ausführungsbeispiel der beanspruchten Zusammensetzung.

Zur Erhebung der klinischen Versuchsdaten wurden 80 Probanden in eine Gruppe zur Einnahme der Kaugummies gemäß dem Ausführungsbeispiel der beanspruchten Zusammensetzung mit 40 Probanden und in eine Gruppe zur Einnahme der Tabletten gemäß der alternativen Zusammensetzung mit 40 Probanden aufgeteilt. Je nach Gruppe nahm der Proband über einen Zeitraum von 14 Tagen zweimal am Tag einen Kaugummi gemäß dem Ausführungsbeispiel der beanspruchten Zusammensetzung beziehungsweise zwei Tabletten gemäß der alternativen Zusammensetzung ein.

Zur Erhebung der Versuchsdaten füllten die Probanden am ersten Tag, am dritten Tag, am fünften Tag, am achten Tag, am elften Tag und am vierzehnten Tag je einen Fragebogen nach dem Brief Mood Introspection Scale, kurz BMIS, 3rd edition, February 26, 2019 aus, mit dem sie auf verschiedenen Skalen, Angaben zu Ihrer Stimmung machten.

Mit jedem Fragebogen wurde die Stimmung eines Proband auf sechzehn verschiedenen Stimmungsskalen erhoben. Die einzelnen Stimmungsskalen sind durch die BMIS vorgegeben und eine Stimmungsskala mit der gefragt wird, wie lebendig (in BMIS: "*Lively*") sich der Proband fühlt, eine Stimmungsskala, wie glücklich (in BMIS: *"Happy"*) sich der Proband fühlt, eine Stimmungsskala, wie traurig (in BMIS: *"Sad*") sich der Proband fühlt, eine Stimmungsskala, wie müde (in BMIS: "*Tired*") sich der Proband fühlt, eine Stimmungsskala, wie emotional aufmerksam (in BMIS: "*Caring*") sich der Proband fühlt, eine Stimmungsskala, wie zufrieden (in BMIS: *"Content*") sich der Proband fühlt, eine Stimmungsskala, wie bedrückt (in BMIS: "*Gloomy*") sich der Proband fühlt, eine Stimmungsskala, wie überspannt (in BMIS: "*Jittery*") sich der Proband fühlt, eine Stimmungsskala, wie schläfrig (in BMIS: "*Drowsy*") sich der Proband fühlt, eine Stimmungsskala, wie muffelig (in BMIS: "*Grouchy*") sich der Proband fühlt, eine Stimmungsskala, wie schwungvoll (in BMIS: "*Peppy*") sich der Proband fühlt, eine Stimmungsskala, wie nervös (in BMIS: "*Nervous*") sich der Proband fühlt, eine Stimmungsskala, wie ruhig (in BMIS: "*Calm*") sich der Proband fühlt, eine Stimmungsskala, wie liebevoll (in BMIS: "*Loving*") sich der Proband fühlt, eine Stimmungsskala, wie überfordert (in BMIS: "*Fed up")* sich der Proband fühlt und eine Stimmungsskala, wie tätig (in BMIS: "*Active*") sich der Proband fühlt.

Auf jeder Skala konnte sich der Proband entscheiden, ob die Stimmung der entsprechenden Skala für ihn sicher zutraf, ob die Stimmung der entsprechenden Skala für ihn wahrscheinlich zutraf, ob die Stimmung der entsprechenden Skala für ihn wahrscheinlich nicht zutraf oder ob die Stimmung der entsprechenden Skala für ihn sicher nicht zutraf. Traf die Stimmung für den Probanden sicher zu, wurde die Entscheidung des Probanden mit dem Wert 4 gewertet. Traf die Stimmung für den Probanden wahrscheinlich zu, wurde die Entscheidung des Probanden mit dem Wert 3 gewertet. Traf die Stimmung für den Probanden wahrscheinlich nicht zu, wurde die Entscheidung des Probanden mit dem Wert 2 gewertet. Traf die Stimmung für den Probanden sicher nicht zu, wurde die Entscheidung des Probanden mit dem Wert 1 gewertet.

Die Fragebögen wurden ausgewertet, indem zunächst für jede Gruppe über alle Teilnehmer für jeden Tag und für jede Skala der Mittelwert gebildet wurde.

In der nachstehenden Tabelle 1 ist das Ergebnis dieser Mittelwertbildung für die Gruppe dargestellt, denen die Kaugummies gemäß dem Ausführungsbeispiel der beanspruchten Zusammensetzung verabreicht wurde:

**(Tabelle 1)**

| | Tag 1 | Tag 3 | Tag 5 | Tag 8 | Tag 11 | Tag 14 |
|---|---|---|---|---|---|---|
| Lively | 2,63 | 2,68 | 2,93 | 2,98 | 2,98 | 2,93 |
| Happy | 2,78 | 2,70 | 2,85 | 2,97 | 3,03 | 2,88 |
| Sad | 1,80 | 2,03 | 1,93 | 1,83 | 1,68 | 1,80 |
| Tired | 2,48 | 2,38 | 2,15 | 2,08 | 2,15 | 2,08 |
| Caring | 2,65 | 2,73 | 2,73 | 2,85 | 2,83 | 2,85 |
| Content | 2,78 | 2,78 | 2,83 | 2,95 | 2,98 | 3,08 |
| Gloomy | 1,90 | 2,00 | 1,80 | 1,78 | 1,83 | 1,65 |
| Jittery | 1,63 | 1,55 | 1,73 | 1,63 | 1,55 | 1,60 |
| Drowsy | 2,20 | 2,38 | 2,10 | 2,05 | 2,03 | 2,08 |
| Grouchy | 1,63 | 1,65 | 1,70 | 1,50 | 1,68 | 1,55 |
| Peppy | 2,48 | 2,40 | 2,60 | 2,63 | 2,78 | 2,73 |
| Nervous | 1,93 | 2,03 | 1,60 | 1,70 | 1,75 | 1,65 |
| Calm | 2,60 | 2,70 | 2,90 | 2,83 | 3,05 | 3,08 |
| Loving | 2,78 | 2,75 | 2,80 | 2,95 | 2,98 | 2,93 |
| Fed up | 2,00 | 2,18 | 1,90 | 1,80 | 1,88 | 1,78 |
| Active | 2,53 | 2,53 | 2,78 | 2,85 | 2,80 | 2,80 |

Demgegenüber ist in der nachstehenden Tabelle 2 das Ergebnis der Mittelwertbildung für die Gruppe dargestellt, denen die Tabletten gemäß der alternativen Zusammensetzung verabreicht wurde:

**(Tabelle 2)**

| | Tag 1 | Tag 3 T | Tag 5 | Tag 8 | Tag 11 | Tag 14 |
|---|---|---|---|---|---|---|
| Lively | 2,78 | 2,83 | 2,95 | 3,08 | 2,98 | 3,20 |
| Happy | 2,88 | 2,95 | 3,13 | 3,00 | 2,88 | 3,13 |
| Sad | 1,70 | 1,73 | 1,63 | 1,73 | 1,88 | 1,60 |
| Tired | 2,25 | 2,13 | 1,85 | 2,00 | 2,00 | 1,83 |
| Caring | 2,70 | 2,80 | 3,00 | 2,80 | 2,83 | 2,90 |
| Content | 2,88 | 2,95 | 3,18 | 3,25 | 2,98 | 3,15 |
| Gloomy | 1,63 | 1,75 | 1,75 | 1,68 | 1,73 | 1,65 |
| Jittery | 1,50 | 1,55 | 1,53 | 1,48 | 1,63 | 1,60 |
| Drowsy | 2,23 | 2,23 | 1,98 | 2,13 | 2,18 | 1,98 |
| Grouchy | 1,68 | 1,60 | 1,45 | 1,55 | 1,53 | 1,48 |
| Peppy | 2,58 | 2,60 | 2,78 | 2,75 | 2,55 | 2,78 |
| Nervous | 1,68 | 1,78 | 1,68 | 1,78 | 1,80 | 1,73 |
| Calm | 2,80 | 2,95 | 3,05 | 3,00 | 2,98 | 3,10 |
| Loving | 2,83 | 2,98 | 3,05 | 3,00 | 2,95 | 3,03 |
| Fed up | 1,90 | 1,80 | 1,80 | 1,89 | 1,80 | 1,76 |
| Active | 2,63 | 2,78 | 2,93 | 2,83 | 2,73 | 2,80 |

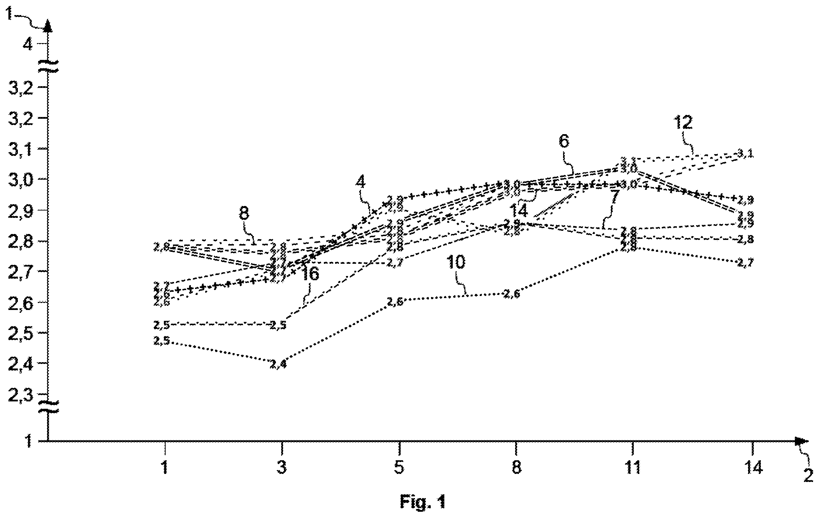

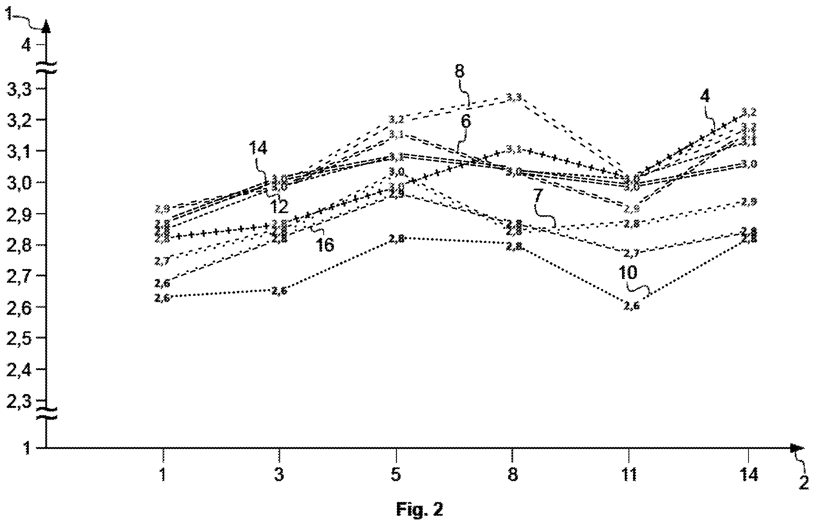

Die Werte 1 aus den Tabellen 1 und 2 sind in den Figs. 1 bis 4 über die Zeit 2 aufgetragen. Dabei zeigt die Fig. 1 die Werte 1 der positiv konnotierten Stimmungskalen der Tabelle 1. Hierbei handelt es sich um die Stimmungsskalen lebendig 4 (für Lively), glücklich 6 (für Happy), emotional aufmerksam 7 (für Caring), zufrieden 8 (für Content), schwungvoll 10 (für Peppy), ruhig 12 (für Calm), liebevoll 14 (für Loving) und tätig 16 (für Active). In der Fig. 2 sind die gleichen positiv konnotierten Stimmungskalen aufgetragen, jedoch aus Tabelle 2.

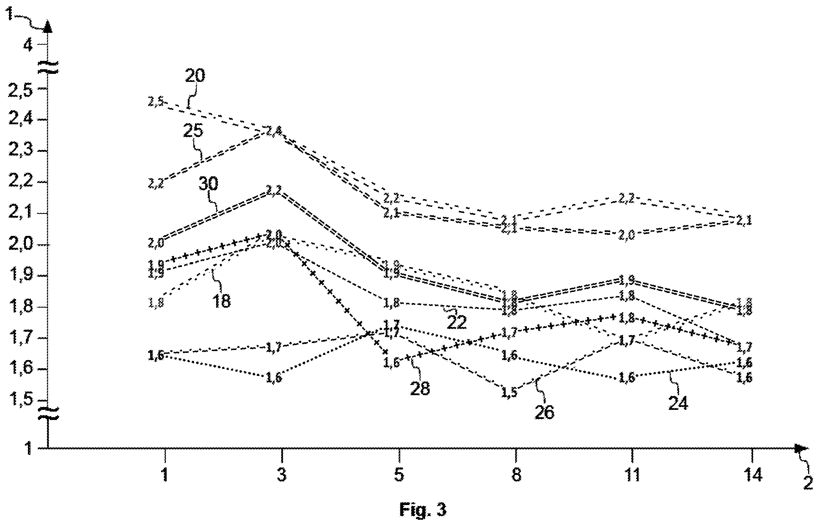

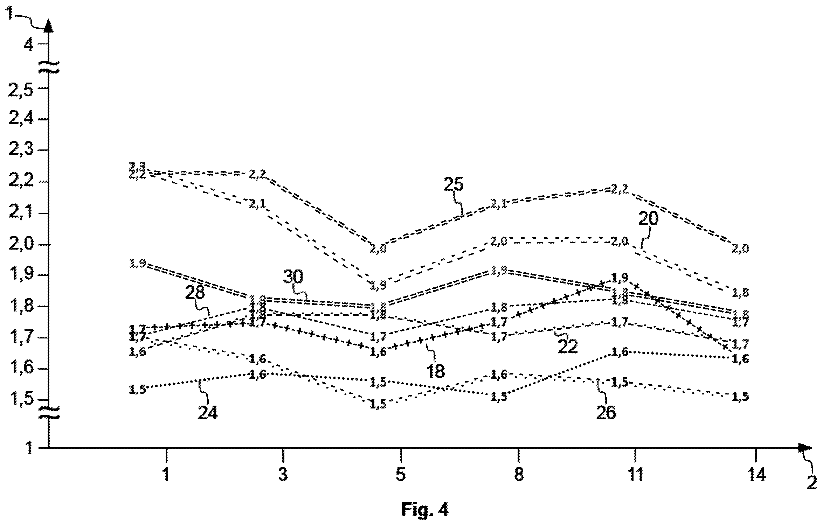

Die Fig. 3 zeigt die Werte 1 der negativ konnotierten Stimmungskalen der Tabelle 1. Hierbei handelt es sich um die Stimmungsskalen traurig 18 (für Sad), müde 20 (für Tired), bedrückt 22 (für Gloomy), überspannt 24 (für Jittery), schläfrig 25 (für Drowsy), muffelig 26 (für Grouchy), nervös 28 (für Nervous) und überfordert 30 (für Fed up). In der Fig. 4 sind die gleichen negativ konnotierten Stimmungskalen aufgetragen, jedoch aus Tabelle 2.

Die Fig. 1 und 2 zeigen, dass alle stimulierenden Stimmungsskalen einen positiven Trend zeigen, sowohl bei der beanspruchten Zusammensetzung, als auch bei der alternativen Zusammensetzung. Signifikant in der Einzelauswertung ist bei der beanspruchten Zusammensetzung die Stimmungsskala ruhig 12 für "*Calm"* und bei der alternativen Zusammensetzung die Stimmungsskala lebendig 4 für "*Lively*". Poolt man die Probandengruppen beider Zusammensetzungen ergibt sich noch zusätzlich eine signifikante Verbesserung für die Stimmungsskala zufrieden 8 für "*Satisfied*".

Die in den Figs. 1 und 2 zu den Stimmungsskalen gehörenden Stimmungen werden als angenehm und stimulierend und positiv empfunden, wie dies in Tabelle 1.1 der Brief Mood Introspection Scale, kurz BMIS, 3rd edition, February 26, 2019 dargestellt ist. All diese positiven Stimmungen konnten mit der beanspruchten Zusammensetzung gleichermaßen wie in der alternativen Zusammensetzung im Trend oder signifikant gesteigert werden.

Die Fig. 3 und 4 zeigen, dass alle hemmenden Stimmungsskalen einen konstanten Trend zeigen, sowohl bei der beanspruchten Zusammensetzung, als auch bei der alternativen Zusammensetzung. Signifikant in der Einzelauswertung ist bei der beanspruchten Zusammensetzung sogar ein abnehmender Trend der Müdigkeit 20, der bei der alternativen Zusammensetzung so nicht erkennbar ist. Die Probanden waren wacher und konnten sich besser konzentrieren.

Anschließend wurden zur weiteren Bewertung aus den Mittelwerten für jeden Tag ein Wert auf den vier Unterskalen nach BMIS berechnet, also ein Tageswert auf der Zufriedenheitsskala (nach BMIS: "Pleasent-Unpleasant"), ein Tageswert auf der Erregungsskala (nach BMIS: "Arousal-Calm"), ein Tageswert auf der Müdigkeitsskala (nach BMIS: "Positive-Tired") und ein Tageswert auf der Entspanntheitsskala (nach BMIS: "Negative-Relaxed").

Die nachstehende Tabelle 3 zeigt die Werte für die Unterskalen für die Gruppe, denen die Kaugummies gemäß dem Ausführungsbeispiel der beanspruchten Zusammensetzung verabreicht wurde:

**(Tabelle 3)**

| | Tag 1 | Tag 3 | Tag 5 | Tag 8 | Tag 11 | Tag 14 |
|---|---|---|---|---|---|---|
| Pleasant - Unpleasant | 5,7 | 5,1 | 7,5 | 8,65 | 8,9 | 9,1 |
| Excited - Calm | 17,2 | 17,8 | 17,7 | 18,1 | 17,8 | 17,6 |
| Positive - Tired | 8,4 | 8,3 | 9,6 | 10,1 | 10,2 | 10,1 |
| Negative - Relaxed | 6,7 | 7,1 | 6,1 | 5,9 | 5,6 | 5,4 |

Demgegenüber zeigt die nachstehende Tabelle 4 die Werte für die Unterskalen für die Gruppe, denen die Tabletten gemäß der alternativen Zusammensetzung verabreicht wurde:

**(Tabelle 4)**

| | Tag 1 | Tag 3 | Tag 5 | Tag 8 | Tag 11 | Tag 14 |
|---|---|---|---|---|---|---|
| Pleasant - Unpleasant | 7,5 | 8,3 | 10,4 | 9,5 | 8,3 | 10,5 |
| Excited - Calm | 16,9 | 17,5 | 18,2 | 18,0 | 17,9 | 18,1 |
| Positive - Tired | 9,0 | 9,6 | 10,9 | 10,3 | 9,9 | 10,9 |
| Negative - Relaxed | 5,6 | 5,7 | 5,3 | 5,6 | 5,9 | 5,2 |

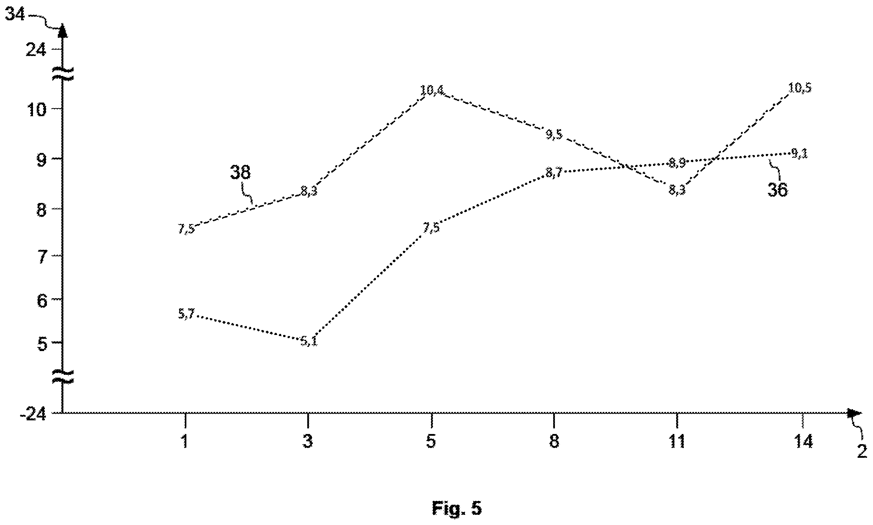

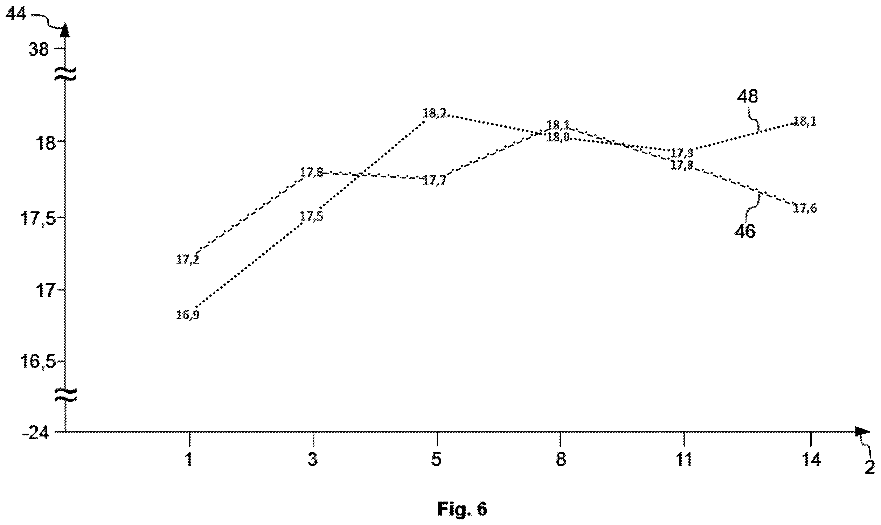

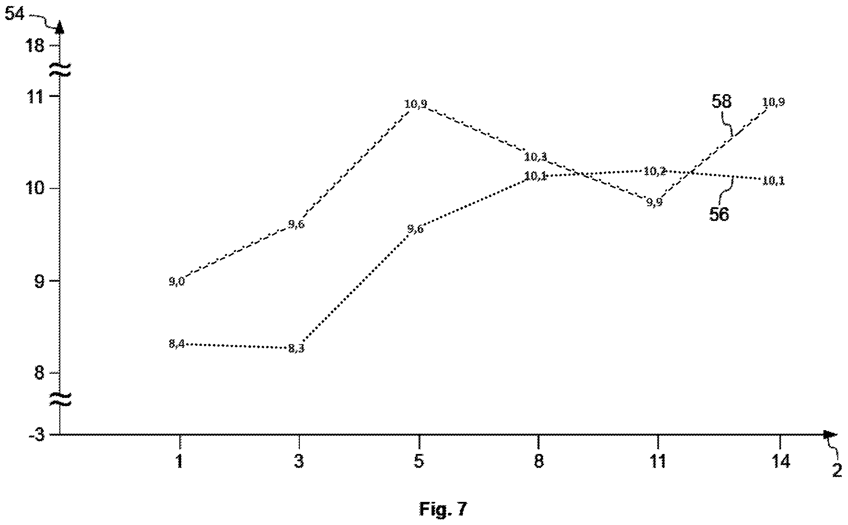

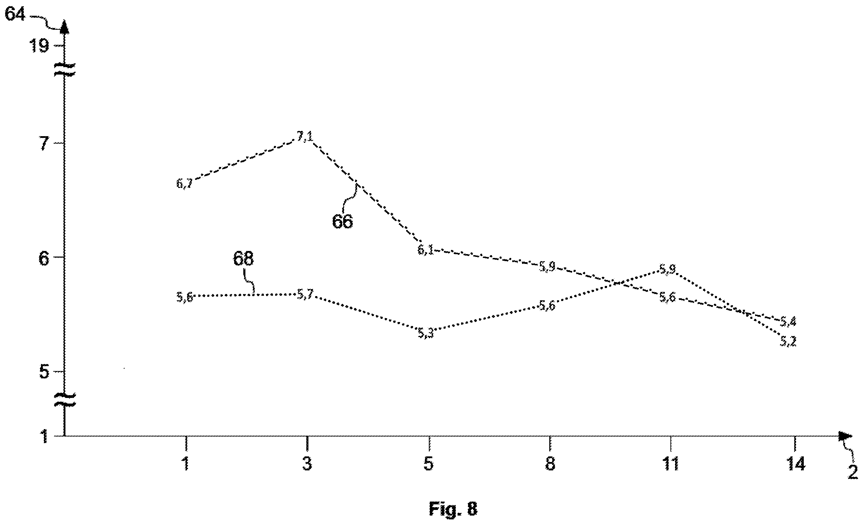

Die so errechneten Werte für die Unterskalen sind zur Bewertung des Ergebnisses in den Diagrammen gemäß Fig. 5 bis 8 über die Zeit 2 aufgetragen. Das Diagramm der Fig. 5 zeigt über die Zeit 2 auf der Zufriedenheitsskala 34 (nach BMIS: "Pleasent-Unpleasant") eine Kaugummi-Zufriedenheitskurve 36 mit den Tageswerten für die Kaugummies gemäß dem Ausführungsbeispiel der beanspruchten Zusammensetzung und eine Tabletten-Zufriedenheitskurve 38 mit den Tageswerten für die Tabletten gemäß der alternativen Zusammensetzung. Das Diagramm der Fig. 6 zeigt über die Zeit 2 auf der Erregungsskala 44 (nach BMIS: "Arousal-Calm") eine Kaugummi-Erregungskurve 46 mit den Tageswerten für die Kaugummies gemäß dem Ausführungsbeispiel der beanspruchten Zusammensetzung und eine Tabletten-Erregungsskurve 48 mit den Tageswerten für die Tabletten gemäß der alternativen Zusammensetzung. Das Diagramm der Fig. 7 zeigt über die Zeit 2 auf der Müdigkeitsskala 54 (nach BMIS: "Positive-Tired") eine Kaugummi-Müdigkeitskurve 56 mit den Tageswerten für die Kaugummies gemäß dem Ausführungsbeispiel der beanspruchten Zusammensetzung und eine Tabletten-Müdigkeitskurve 58 mit den Tageswerten für die Tabletten gemäß der alternativen Zusammensetzung. Das Diagramm der Fig. 8 zeigt über die Zeit 2 auf der Entspanntheitsskala 64 (nach BMIS: "Negative-Relaxed") eine Kaugummi-Entspanntheitskurve 66 mit den Tageswerten für die Kaugummies gemäß dem Ausführungsbeispiel der beanspruchten Zusammensetzung und eine Tabletten-Entspanntheitskurve 68 mit den Tageswerten für die Tabletten gemäß der alternativen Zusammensetzung.

Die Kurven der Figs. 5 bis 8 stützen das aus den Figs. 1 bis 4 interpretierte Ergebnis, dass beide Zusammensetzungen negative Stimmungen senken oder zumindest konstant halten und positive Stimmungen steigern. Die Probanten fühlten sich wacher und waren aufmerksamer.

## Patentansprüche

1. Zusammensetzung, zusammengesetzt aus Lavendelöl als Wirkstoff zur medizinischen Verwendung bei einer Behandlung einer Konzentrationsstörung und einer Kaumasse, **dadurch gekennzeichnet, dass** die Zusammensetzung die Kaumasse umfasst und Lavendelöl als Wirkstoff in einer Konzentration von 0,1 bis 1 Gew %, bevorzugt von 0,2 bis 0,4 Gew %, besonders bevorzugt von 0,3 Gew % liegt, wobei die Kaumasse eine Zugfestigkeit zwischen 0,25 N/mm² und 4 N/mm² zur Beanspruchung der Gesichtsmuskeln aufweist.

2. Zusammensetzung zur Verwendung nach Anspruch 1, wobei das Lavendelöl der einzige Wirkstoff ist.

3. Zusammensetzung zur Verwendung nach einem der vorstehenden Ansprüche, wobei die Kaumasse als Konzistenzbildner synthetischen und/oder natürlichen Gummi aufweist.

4. Zusammensetzung zur Verwendung nach einem der vorstehenden Ansprüche, wobei die Kaumasse 0,05 bis 0,5 Gew.%, vorzugsweise 0,08 bis 0,2 Gew.%, besonders bevorzugt 0,1 Gew% eines Spurenelementes umfasst.

5. Zusammensetzung zur Verwendung nach Anspruch 4, wobei das Spurenelement Zink ist.

6. Zusammensetzung zur Verwendung nach einem der vorstehenden Ansprüche, wobei die Kaumasse 0,1 Gew % bis 1 Gew %, vorzugsweise 0,15Gew % bis 0,35 Gew %, besonders bevorzugt 0,25 Gew % eines Salzes umfasst.

7. Zusammensetzung zur Verwendung nach Anspruch 6, wobei das Salz Natriumchlorid ist.

8. Zusammensetzung zur Verwendung nach einem der vorstehenden Ansprüche, wobei die Kaumasse 0,05 Gew % bis 0,5 Gew %, vorzugsweise 0,09 Gew % bis 0,3 Gew %, besonders bevorzugt 0,15 Gew % eines Vitamin B umfasst.

9. Zusammensetzung zur Verwendung nach Anspruch, wobei das Vitamin B Niacin
und/oder Pantothensäure umfasst.

10. Zusammensetzung zur Verwendung nach einem der vorstehenden Ansprüche, wobei die Kaumasse 0,1 Gew % bis 1 Gew %, vorzugsweise 0,2 Gew % bis 0,4 Gew %, besonders bevorzugt 0,3 Gew % eines Vitamin D umfasst.

11. Zusammensetzung zur Verwendung nach Anspruch 10, wobei das Vitamin D Biotin ist.

12. Zusammensetzung zur Verwendung nach einem der vorstehenden Ansprüche, wobei die Kaumasse ferner einen Säureregulator umfasst.

13. Zusammensetzung zur Verwendung nach Anspruch 12, wobei der Säurerregulator Zitronensäure ist.

14. Zusammensetzung zur Verwendung nach einem der vorstehenden Ansprüche, wobei die Kaumasse einen Zuckeralkohol umfasst.

## Claims

1. Composition, comprising lavender oil as an active ingredient for medicinal use in the treatment of a concentration disorder and a gum base,
**characterized in that** the composition comprises the gum base and lavender oil as the active ingredient in a concentration of 0.1 to 1 wt%, preferably 0.2 to 0.4 wt%, more preferably 0.3 wt%,
wherein the gum base has a tensile strength between 0.25 N/mm² and 4 N/mm² for engaging the facial muscles.

2. Composition for use according to claim 1,
wherein the lavender oil is the only active ingredient.

3. Composition for use according to any one of the preceding claims, wherein the gum base comprises consistency formers of synthetic and/or natural rubber.

4. Composition for use according to any one of the preceding claims, wherein the gum base comprises 0.05 to 0.5 wt%, preferably 0.08 to 0.2 wt%, particularly preferably 0.1 wt% of a trace element.

5. Composition for use according to claim 4,
wherein the trace element is zinc.

6. Composition for use according to any one of the preceding claims,
wherein the gum base comprises 0.1 wt% to 1 wt%, preferably 0.15 wt% to 0.35 wt%, particularly preferably 0.25 wt% of a salt.

7. Composition for use according to claim 6,
wherein the salt is sodium chloride.

8. Composition for use according to any one of the preceding claims, wherein the gum base comprises 0.05 wt% to 0.5 wt%, preferably 0.09 wt% to 0.3 wt%, particularly preferably 0.15 wt% of a vitamin B.

9. Composition for use according to claim 8,
wherein the vitamin B is niacin and/or pantothenic acid.

10. Composition for use according to any one of the preceding claims, wherein the gum base comprises 0.1 wt% to 1 wt%, preferably 0.2 wt% to 0.4 wt%, particularly preferably 0.3 wt% of a vitamin D.

11. Composition for use according to claim 10,
wherein the vitamin D is biotin.

12. Composition for use according to any one of the preceding claims, wherein the gum base further comprises an acidity regulator.

13. Composition for use according to claim 12,
wherein the acidity regulator is citric acid.

14. Composition for use according to any one of the preceding claims, wherein the gum base comprises a sugar alcohol.

## Revendications

1. Composition, comprenant de l'huile essentielle de lavande en tant que principe actif pour une utilisation médicale dans le traitement d'un trouble de la concentration et une base de gomme,
**caractérisée en ce que** la composition comprend la base de gomme et l'huile essentielle de lavande en tant que principe actif à une concentration de 0,1 à 1 % en poids, de préférence de 0,2 à 0,4 % en poids, et plus particulièrement de 0,3 % en poids,
la base de gomme présentant une résistance à la traction comprise entre 0,25 N/mm² et 4 N/mm² pour solliciter les muscles du visage.

2. Composition selon la revendication 1,
dans laquelle l'huile essentielle de lavande est le seul principe actif.

3. Composition selon l'une quelconque des revendications précédentes, dans laquelle la base de gomme comprend un agent de consistance constitué de gomme synthétique et/ou naturelle.

4. Composition selon l'une quelconque des revendications précédentes, dans laquelle la base de gomme comprend de 0,05 à 0,5 % en poids, de préférence de 0,08 à 0,2 % en poids, et plus particulièrement 0,1 % en poids d'un oligo-élément.

5. Composition selon la revendication 4,
dans laquelle l'oligo-élément est du zinc.

6. Composition selon l'une quelconque des revendications précédentes, dans laquelle la base de gomme comprend de 0,1 % en poids à 1 % en poids, de préférence de 0,15 % à 0,35 % en poids, et plus particulièrement 0,25 % en poids d'un sel.

7. Composition selon la revendication 6,
dans laquelle le sel est du chlorure de sodium.

8. Composition selon l'une quelconque des revendications précédentes, dans laquelle la base de gomme comprend de 0,05 % à 0,5 % en poids, de préférence de 0,09 % à 0,3 % en poids, et plus particulièrement 0,15 % en poids d'une vitamine B.

9. Composition selon la revendication 8,
dans laquelle la vitamine B est la niacine et/ou l'acide pantothénique.

10. Composition selon l'une quelconque des revendications précédentes, dans laquelle la base de gomme comprend de 0,1 % à 1 % en poids, de préférence de 0,2 % à 0,4 % en poids, et plus particulièrement 0,3 % en poids d'une vitamine D.

11. Composition selon la revendication 10,
dans laquelle la vitamine D est la biotine.

12. Composition selon l'une quelconque des revendications précédentes, dans laquelle la base de gomme comprend en outre un régulateur d'acidité.

13. Composition selon la revendication 12,
dans laquelle le régulateur d'acidité est l'acide citrique.

14. Composition selon l'une quelconque des revendications précédentes, dans laquelle la base de gomme comprend un polyol.
